(19) Europäisches Patentamt
European Patent Office
Office européen des brevets

(11) **EP 2 766 367 B1**

(12) # EUROPÄISCHE PATENTSCHRIFT

(45) Veröffentlichungstag und Bekanntmachung des Hinweises auf die Patenterteilung:
**18.05.2022 Patentblatt 2022/20**

(21) Anmeldenummer: **12775659.1**

(22) Anmeldetag: **15.10.2012**

(51) Internationale Patentklassifikation (IPC):
***C07D 487/04*** *(2006.01)* ***A61K 31/519*** *(2006.01)*
***A61P 31/12*** *(2006.01)* ***A61P 31/14*** *(2006.01)*
***A61P 31/16*** *(2006.01)*

(52) Gemeinsame Patentklassifikation (CPC):
**C07D 487/04; A61P 31/12; A61P 31/14; A61P 31/16**

(86) Internationale Anmeldenummer:
**PCT/EP2012/070403**

(87) Internationale Veröffentlichungsnummer:
**WO 2013/053942 (18.04.2013 Gazette 2013/16)**

---

(54) **4-Amino-3-phenylamino-6-phenylpyrazolo[3,4-d]pyrimidin-Derivate zur Behandlung von viralen Infekten, insbesondere von Picornavirusinfektionen**

4-Amino-3-phenylamino-6-phenylpyrazolo[3,4-d]pyrimidine derivatives for the treatment of viral infections, in particular picornavirus infections

Dérivés de 4-amino-3-phénylamino-6-phénylpyrazolo[3,4-d]pyrimide pour le traitement des infections virales, notammment des infections des picornaviridés

---

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.10.2011 DE 102011116373**
**20.10.2011 DE 102011116384**

(43) Veröffentlichungstag der Anmeldung:
**20.08.2014 Patentblatt 2014/34**

(73) Patentinhaber: **Scandion Oncology A/S**
**2100 Copenhagen Ø (DK)**

(72) Erfinder:
- **WUTZLER, Peter**
**99101 Erfurt-Windischholzhausen (DE)**
- **SCHMIDTKE, Michaela**
**07743 Jena (DE)**
- **MAKAROV, Vadim**
**Moskau, 115035 (RU)**

(74) Vertreter: **Høiberg P/S**
**Adelgade 12**
**1304 Copenhagen K (DK)**

(56) Entgegenhaltungen:
**WO-A1-94/13677 WO-A1-2007/147401**

Bemerkungen:
Die Akte enthält technische Angaben, die nach dem Eingang der Anmeldung eingereicht wurden und die nicht in dieser Patentschrift enthalten sind.

---

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Die Erfindung wird über die beigefügten Ansprüche definiert. Jegliche Ausführungsform die nicht vom Umfang der beigefügten Ansprüche umfasst wird, ist nicht Teil der Erfindung.

Die Offenbarung betrifft neuartige 4-Amino-3-phenylamino-6-phenylpyrazolo[3,4-d]pyrimidin-Derivate und deren Verwendung als antivirale Wirkstoffe, vorzugsweise zur Verwendung in der Behandlung von Picornavirusinfektionen.

Picornaviren, insbesondere Entero- und Rhinoviren, sind für ein breites Spektrum von Erkrankungen beim Menschen verantwortlich. Zu den Enteroviren gehören mehr als 60 verschiedene humanpathogene Serotypen (Melnick J in: Fields B et al., editors. Virology. Philadelphia: Lippincott-Raven Publishers; 1996, 655-712). Enterovirus-, Echovirus-, Coxsackievirus A- und B-Infektionen verlaufen oftmals mit unspezifischem Fieber und rufen Erkrankungen des oberen Respirationstraktes hervor, die sich häufig nicht von Rhinovirusinfektionen unterscheiden lassen. Zu den schwereren Krankheitsbildern, die auch epidemisch auftreten können, gehören die hämorrhagische Konjunktivitis, Herpangina, Hand-Fuß-Mund-Krankheit, aseptische Meningitis, Enzephalitis und akute Myokarditis. Dabei können verschiedene Virustypen gleiche Symptome bzw. ein Virustyp ganz unterschiedliche Krankheitsbilder herbeiführen. Mit der Einführung moderner und sensitiver Methoden in die Virusdiagnostik gelang der Nachweis persistierender enteroviraler RNA sowie von Virusproteinen im Zusammenhang mit chronischen Erkrankungen wie z. B. dem Typ II Diabetes, Poliomyositis und vor allem der chronischen Myokarditis. Persistierende Enterovirusinfektionen kommen auch bei Patienten mit Agammaglobulinämie vor und äußern sich hierbei als persistierende Enterovirus-Meningoenzephalitis. Als Begleiterscheinungen traten häufig eine Dermatomyositis oder Polymyositis auf. Zu den Rhinoviren gehören ca. 100 Serotypen. Rhinovirusinfektionen verursachen mehr als die Hälfte aller respiratorischen Erkrankungen des oberen Respirationstraktes beim Menschen (Couch RB in: Fields BM et al., editors: Fields Virology, 3rd edition. Lippincott-Raven, Philadelphia, 1996, 713-35). Bei einer mittleren Krankheitsdauer von ca. 10 Tagen führen diese meist harmlos verlaufenden Erkältungen jährlich zu millionenfachen Arztbesuchen, Arbeits- und Schulausfällen. Als Komplikationen können eine Otitis media, Sinusitis, Exazerbation von Asthma und zystischer Fibrose sowie Infekte des unteren Respirationstraktes vor allem bei Kleinkindern, älteren Patienten und immunsupprimierten Patienten auftreten. Auf Grund der Typenvielfalt ist eine Impfprophylaxe zurzeit nicht möglich. Bedingt durch die mit diesen Erkrankungen verbundenen Arbeitsausfälle, Arztbesuche und Medikamente verursachen Rhino- und Enteroviren jährlich enorme Kosten. Die Behandlung dieser Virusinfektionen erfolgt bisher symptombezogen, da keine virusspezifischen Therapeutika zur Verfügung stehen (Rotbart HA: Antiviral Res 2002, 53(2), 83-98). Zudem werden häufig unnütz Antibiotika verordnet. Die Entwicklung von neuen Virustatika ist deshalb unbedingt notwendig.

Die Ergebnisse der intensiven Suche nach Behandlungsmöglichkeiten von Enterovirus- und Rhinovirus-Infektionen wurden von Rotbart 2002 in einem Übersichtsartikel zusammengefasst (Rotbart HA: Antiviral Res 2002, 53(2), 83-98). Beispielsweise hemmt Ribavirin ein Wirtszellenzym, die Inosin 5'-Monophosphat (IMP)-Dehydrogenase. Durch die Ausschaltung dieses Schlüsselenzymes für die Synthese von Purinnukleotiden, lässt sich die Replikation von Picornaviren *in vitro* und *in vivo* hemmen. Ribavirin soll außerdem direkt in das Genom von Polioviren eingebaut werden und dadurch zusätzlich als Mutagen für RNA-Viren wirken (Crotty S et al.: Nat Med, 2000, 6(12),1375-9). Wegen starker Nebenwirkungen werden diese Verbindungen nicht zur Behandlung von Rhino- und Enterovirusinfektionen eingesetzt. Spezifische Targets zur Hemmung der viralen RNA-Synthese stellen das Genom an sich, die virale RNA-abhängige RNA-Polymerase sowie weitere für den Replikationskomplex notwendige virale Proteine dar. Guanidine, Thiosemicarbazone, Benzimidazole, Dipyridamole und Flavone sind seit langem als Inhibitoren der Polymerasen von verschiedenen Picornaviren in der Zellkultur bekannt. Sehr unterschiedliche Erfolge wurden damit *in vivo* erzielt. Als aussichtsreichster Kandidat mit breiter anti-Enterovirus- und anti-Rhinovirus-Aktivität gelten Enviroximderivate. Enviroxim verhindert die Synthese von Plusstrang RNA durch Bindung an das Virusprotein 3A, welches zur Bildung von RNA- Intermediaten bei der Virusvermehrung notwendig ist (Heinz BA und Vance LM: J Virol, 1995, 69(7), 4189-97). In klinischen Studien wurden moderate oder keine therapeutische Wirkungen, eine schlechte Pharmakokinetik und unerwünschte Nebenwirkungen festgestellt (Miller FD et al.: Antimicrob Agents Chemother, 1985, 27(1), 102-6). Von neueren Derivaten mit besserer Bioverfügbarkeit und Verträglichkeit liegen bisher keine klinischen Daten vor.

Basierend auf dem Wissen der Feinstruktur und Funktion der viralen Protease 2C wurde der Proteaseinhibitor AG 7088 entwickelt. AG 7088 wirkt in der Zellkultur im nanomolaren Konzentrationsbereich gegen 48 Rhinovirustypen sowie Coxsackievirus A21, B3, Enterovirus 70 und Echovirus 11 (Pattick AK et al.: Antimicrobila Agents Chemother, 1999, 43(10), 2444-50). Bisher sind die abschließenden Daten der klinischen Studien nicht bekannt.

Mit der Aufklärung der molekularen Struktur der viralen Kapside wurden die Voraussetzungen für ein zielgerichtetes Design von Kapsidblockern, den "WIN-Substanzen", geschaffen (Diana GD: Curr Med Chem 2003, 2, 1-12). Sie verhindern die Adsorption und/oder das Uncoating von Rhino- und Enteroviren. Einige der WIN-Substanzen wirken hochspezifisch nur gegen einzelne Gattungen oder Virustypen der Picornaviren. Andere Derivate hemmen die Vermehrung von Rhinoals auch Enteroviren. Zu den WIN-Substanzen gehören z. B. Arildone, Disoxaril und Pirodavir. Diese Verbindungen zeigten sehr gute antivirale Wirkungen in der Zellkultur. Eine schlechte Löslichkeit (Arildone), niedrige Bioverfügbarkeit (Arildone und Disoxaril), schnelle Metabolisierung und Ausscheidung (Disoxaril und WIN 54954) sowie

Nebenwirkungen, wie beispielsweise Hautausschlag (WIN 54954), machten eine klinische Anwendung unmöglich. Große Hoffnungen wurden auf Pleconaril, einen weiteren Kapsidblocker, gesetzt. Pleconaril besitzt eine sehr gute orale Bioverfügbarkeit und hemmt nach seiner Bindung in die hydrophobe Tasche im Viruskapsid die Penetration von Rhino-, Echo- und Coxsackieviren (Pevear DC et al.: Antimicrob Agents Chemother 1999, 43(9), 2109-15; McKinlay MA et al.: Annu Rev Microbiol 1992, 46, 635-54). Dadurch ist es potenziell wirksam gegen ein breites Spektrum von Viruserkrankungen, von der gewöhnlichen Erkältung bis hin zur viralen Meningitis oder Myokarditis. Resistenzen wurden bei Rhinoviren, Enterovirus 71 und Coxsackievirus B3 beobachtet (Ledford RM et al.: J Virol 2004, 78(7), 3663-74; Groarke JM et al.: J Infect Dis 1999, 179(6), 1538-41). Klinische Studien bei Kindern und Erwachsenen mit einer Enterovirusmeningitis (Abzug MJ et al.: Pediatr Infect Dis J, 2003, 22, 335-41) sowie Rhinovirus-verursachten respiratorischen Infektionen (Hayden FG et al.: Antivir Ther, 2002, 7, 53-65; Hayden FG et al.: Clin Infect Dis, 2003, 36, 1523-32) verliefen positiv. Die nachgewiesene therapeutische Wirkung reichte jedoch nicht für die Zulassung von Pleconaril (Picovir, Viropharma, USA) zur Behandlung von Rhinovirusinfektionen in den USA. Im März 2002 wurde ein entsprechender Antrag von der Nahrungs- und Arzneimittel-Behörde (Food and Drug Administration: FDA) wegen einer ungünstigen Nutzen-Risiko-Bewertung abgelehnt.

Es sind auch Pyrazolopyrimidine als CRF-Antagonisten (Corticotropin-Releasing Factor Antagonisten) beschrieben (z. B. EP 674 642 und EP 691 128), welche beispielsweise die Adenosinkinase (EP 496 617 oder US 4,904,666), die Xanthinoxigenase (J. Heterocyc. Chem. 19, 1565, 1982) oder andere Enzymsysteme (US 2,965,643 und US 3,600,389) hemmen.

Somit besteht weiterhin als eine dringliche Aufgabe der antiviralen Forschung die Entwicklung hocheffektiver Virustatika zur Behandlung von Rhino- und Enteroviruserkrankungen. Die neuen Verbindungen sollten gut verträglich sein und existierende Resistenzen, z. B. gegenüber Pleconaril, überwinden.

WO 00/43394 A offenbart substituierte Pyrazolo[3,4-d]pyrimidin-Derivate und deren Verwendung als antivirale Wirkstoffe.

[Seite 4a]

Auch EP 2 049 540 (WO 2007/147401) offenbart 4-Amino-3-arylamino-6-arylpyrazolo[3,4-d]pyrimidin-Derivate und deren Verwendung als antivirale Wirkstoffe.

Aufgabe der Offenbarung ist es, weitere Pyrazolo[3,4-d]pyrimidin-Derivate sowie deren Herstellung und Verwendung bei der prophylaktischen oder therapeutischen Behandlung von Enteroviren und Rhinoviren sowie die angegebenen Nachteile des Standes der Technik zum Beispiel hinsichtlich Stabilität und Bioverfügbarkeit der Substanzen vermeiden.

Gelöst wird diese Aufgabe durch 4-Amino-3-phenylamino-6-phenylpyrazolo[3,4-d]pyrimidin-Derivate der allgemeinen Formel IIa

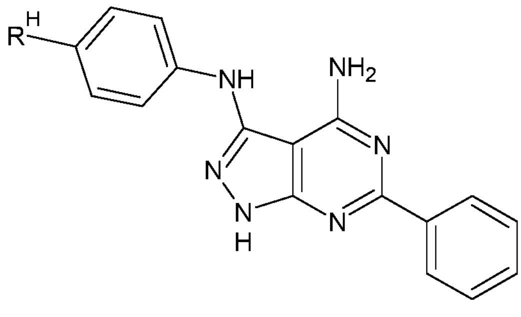

IIa,

oder IIb

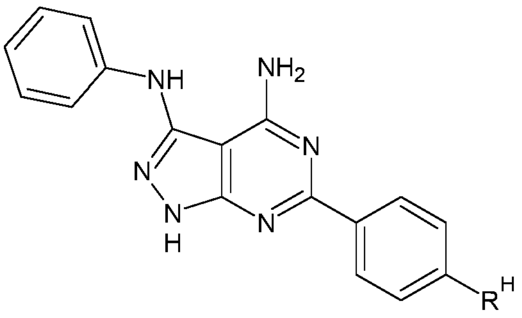

IIb,

wobei $R^H$ ausgewählt ist aus $NO_2$, CN, $CF_3$, $CCl_3$, CBr3, $OCF_3$, $OCCl_3$, $OCBr_3$, $CHF_2$, $CHCl_2$, $CHBr_2$, $OCHCl_2$, CHO, COOH, COMe, COEt, COOMe oder COOEt ist, bevorzugt $CF_3$ oder $OCF_3$.

In den Unteransprüchen sind vorteilhafte Ausführungen der vorliegenden Offenbarung der spezifisch substituierten 4-Amino-3-phenylamino-6-phenylpyrazolo[3,4-d]pyrimidin-Derivate sowie Anwendungsmöglichkeiten ausgeführt, ohne die Offenbarung jeweils darauf zu beschränken.

Offenbart sind weiterhin 4-Amino-3-phenylamino-6-phenylpyrazolo[3,4-d]pyrimidin-Derivate der allgemeinen Formel I, einschließlich ihrer pharmazeutisch verträglichen Salzverbindungen und ihrer Pro-Pharmaka,

Bei den Arylsubstituenten in 3'- und 6'-Position kann es sich gemäß der EP 2 049 540 um Phenyl oder Naphthyl-Substituenten, aber auch um verschiedenste aromatische Heterocyclen mit ein oder mehreren Stickstoff, Schwefel oder Sauerstoffatomen handeln. Diese Arylsubstituneten können zudem eine Vielzahl von Substituenten wie Halogenen, Alkylresten oder Alkoxresten aufweisen. Die beschriebenen Verbindungen sollen insbesondere für die Behandlung von Picornavireninfektionen verwendbar sein.

Die WO 94/13677 offenbart Corticotropin-release Faktor (CRF) Antagonisten auf Basis von Pyrazolo[3,4-d]pyrimidin-Grundkörpern, die in 6'-Position allerdings keinen Phenylsubstitueneten und in 3'-Position keinen Aminophenylsubstituenten aufweisen. Die in der WO 94/13677 beschriebenen Verbindungen sollen insbesondere zur Behandlung von Entzündungserkrankungen, Depressionen und Erkrankungen im Zusammenhang mit Angstzuständen geeignet sein.

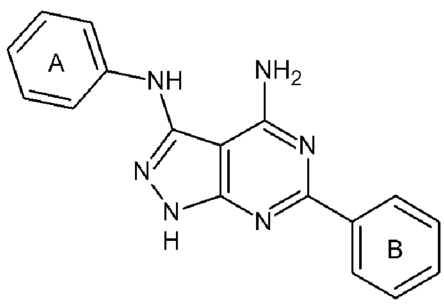

I

wobei zumindest ein Wasserstoffatom in zumindest einer der Phenylgruppen A und B durch einen Substituenten $R^H$ ersetzt ist, der eine Hammett-Konstante $\sigma_p$ größer als 0,23 aufweist,

wobei jedes weitere Wasserstoffatom in jeder der Phenylgruppen A und B unabhängig voneinander ersetzt sein kann durch einen Rest $R^1$, wobei

jedes $R^1$ unabhängig ein Halogen, ein gesättigtes oder ungesättigtes, lineares oder verzweigtes aliphatisches Radikal mit 1-7 Kettengliedern, ein gesättigtes oder ungesättigtes, lineares oder verzweigtes alkanoles Radikal mit 1-8 Kettengliedern, $NO_2$, CN, $CONR^2{}_2$, $COR^2$, $COOR^2$, $OR^2$, $SR^2$, $NR^2{}_2$, $SO_2NR^2{}_2$, $CX_3$, $CR^2X_2$, $OCX_3$, $OCR^2X_2$, oder Phenyl sein kann;

jedes $R^2$ unabhängig Wasserstoff, ein gesättigtes oder ungesättigtes, halogeniertes oder nicht halogeniertes, lineares oder verzweigtes aliphatisches Radikal mit 1-7 Kettengliedern, Benzyl, Phenyl oder Naphtyl, ein gesättigter oder ungesättigter, Mono- oder Polyheterozyklus mit den Heteroatomen N, S oder O ist, wobei jede der vorstehenden genannten Gruppen unabhängig mit Fluor, Chlor, Brom, Trifluormethyl, Alkyl, Alkoxy, Cyano, Nitro, Amino, Aminoalkyl, C(O)-Alkyl, C(O)O-Alkyl, Benzyl, Phenyl oder Naphtyl substituiert sein kann; und

X unabhängig F, Cl, Br, oder I ist.

In den 4-Amino-3-phenylamino-6-phenylpyrazolo[3,4-d]pyrimidin-Derivaten der allgemeinen Formel I kann zumindest ein Wasserstoffatom in zumindest einer der Phenylgruppen A und B durch einen Substituenten $R^H$ ersetzt sein, ausgewählt aus $NO_2$, CN, $CF_3$, $CCl_3$, $CBr_3$, $OCF_3$, $OCCl_3$, $OCBr_3$, $CHF_2$, $CHCl_2$, $CHBr_2$, $OCHCl_2$, CHO, COOH, COMe, COEt, COOMe oder COOEt, bevorzugt $CF_3$ oder $OCF_3$. In einer oder beiden Phenylgruppen A und B können ein, zwei oder drei Wasserstoffatome durch einen Substituenten $R^H$ ersetzt sein. Zudem kann genau ein Wasserstoffatom in einer der Phenylgruppen A und B durch einen Substituenten $R^H$ ersetzt sein. Der Substituent $R^H$ kann sich in para-Position des Phenylrings A oder B befinden.

Zusätzlich zu $R^H$ kann jede der Phenylgruppen A und B unabhängig voneinander weitere Reste $R^1$ tragen. Die Phenylgruppen A und B können unabhängig voneinander keinen, einen, zwei oder drei weitere Reste $R^1$, insbesondere keinen oder einen weiteren Rest $R^1$ tragen.

Als Alkyle kommen insbesondere lineare oder verzweigte $C_{1-7}$-Alkyle, zum Beispiel Methyl, Ethyl, n-Propyl, i-Propyl und Butyl infrage. Entsprechendes gilt für Alkanole, Alkylamine und Alkylamide.

Offenbart sind weiterhin 4-Amino-3-phenylamino-6-phenylpyrazolo[3,4-d]pyrimidin-Derivate der allgemeinen Formel II

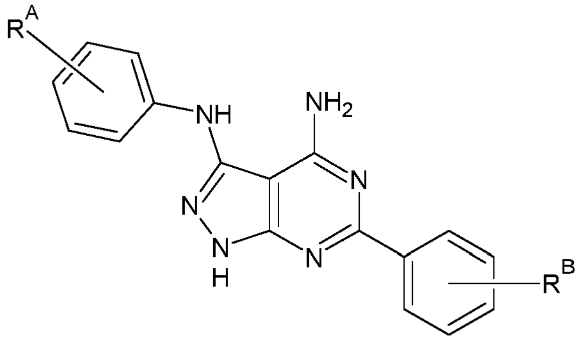

II

wobei jeder Substituent $R^A$, $R^B$ unabhängig Wasserstoff, ein Halogen, ein gesättigtes oder ungesättigtes, lineares oder verzweigtes aliphatisches Radikal mit 1-7 Kettengliedern, ein gesättigtes oder ungesättigtes, lineares oder verzweigtes alkanoles Radikal mit 1-8 Kettengliedern, $NO_2$, CN, $CONR^2_2$, $COR^2$, $COOR^2$, $OR^2$, $SR^2$, $NR^2_2$, $SO_2NR^2_2$, $CX_3$, $CR^2X_2$, $OCX_3$, $OCR^2X_2$, oder Phenyl sein kann; und $R^2$ und X wie oben definiert sind; wobei zumindest einer der Substituenten $R^A$, $R^B$ eine Hammett-Konstante $\sigma_p$ größer 0,23 aufweist.

Die Offenbarung umfasst 4-Amino-3-phenylamino-6-phenylpyrazolo[3,4-d]pyrimidin-Derivate der allgemeinen Formel IIa

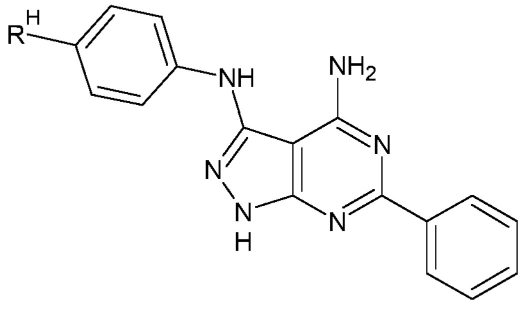

IIa,

oder IIb

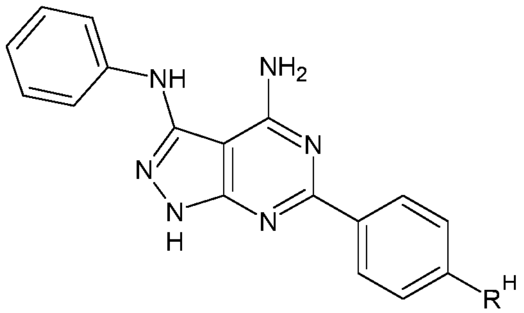

IIb,

wobei $R^H$ ausgewählt ist aus $NO_2$, CN, CF3, $CCl_3$, $CBr_3$, $OCF_3$, $OCCl_3$, $OCBr_3$, $CHF_2$, $CHCl_2$, $CHBr_2$, $OCHCl_2$, CHO, COOH, COMe, COEt, COOMe oder COOEt ist, bevorzugt $CF_3$ oder $OCF_3$.

Ebenfalls offenbart sind pharmazeutische Zusammensetzungen, die ein 4-Amino-3-phenylamino-6-phenylpyrazolo[3,4-d]pyrimidin-Derivat gemäß der allgemeinen Formeln IIa oder IIb enthalten. Solche pharmazeutischen Zusammensetzungen können weitere Substanzen enthalten, beispielsweise pharmazeutisch akzeptable Hilfsstoffe und Träger. In einem besonderen Aspekt der vorliegenden Offenbarung können die pharmazeutischen Zusammensetzungen weitere Wirkstoffe umfassen, insbesondere antivirale Wirkstoffe, vor allem Wirkstoffe gegen Picornaviren.

Es hat sich überraschend gezeigt, dass die 4-Amino-3-phenylamino-6-phenylpyrazolo[3,4-d]pyrimidin-Derivate der vorliegenden Offenbarung über eine deutlich verbesserte Stabilität in Lebermikrosomen gegenüber den Substanzen des Standes der Technik verfügen. Darüber hinaus haben Untersuchungen zur Pharmakokinetik in der Maus ergeben, dass die 4-Amino-3-phenylamino-6-phenylpyrazolo[3,4-d]pyrimidin-Derivate der vorliegenden Offenbarung eine deutlich bessere Bioverfügbarkeit aufweisen als Substanzen des Standes der Technik. Gleichzeitig zeigen die Verbindungen der vorliegenden Offenbarung eine starke antivirale Aktivität gegenüber Picornaviren, insbesondere Entero- und Rhinoviren im nano- bzw. mikromolaren Konzentrationsbereich.

Daher sind die pharmazeutischen Zubereitungen, die eine Verbindung der Formeln IIa oder IIb enthalten, besonders geeignet für die Behandlung von respiratorischen Infekten, der aseptischen Meningitis, der Enzephalitis, Herpangina usw. bei Mensch und Tier, die von Picornaviren insbesondere Entero- und Rhinoviren hervorgerufen werden können.

Die vorstehend offenbarten 4-Amino-3-phenylamino-6-phenylpyrazolo[3,4-d]pyrimidin-Derivate tragen an einer oder an beiden Phenylgruppen mindestens einen Substituenten $R^H$, der eine Hammett-Konstante $\sigma_p$ größer als 0,23 aufweist. Dieser Wert 0,23 entspricht der Hammett-Konstante $\sigma_p$ des Broms, das unter den Halogenen die höchste Hammett-Konstante für die para-Position zeigt.

Die Bestimmung der Hammett-Konstanten für verschiedene Substituenten basiert auf den Ionisierungskonstanten der Benzoesäure gemäß der Hammett-Gleichung

$$\sigma_x = \log K_X - \log K_H$$

wobei $K_H$ die Ionisierungskonstante für Benzoesäure in Wasser bei 25 °C ist und $K_x$ die korrespondierende Konstante für eine meta- oder para-substituierte Benzoesäure ist. Ein Verfahren zur Bestimmung der Hammett-Konstante für verschiedene Substituenten in meta- ($\sigma_m$) und para-Position ($\sigma_p$) sowie bereits ermittelte Werte einer Vielzahl von Substituenten können der Veröffentlichung von Hansch et al., "A Survey of Hammett Substituent Constants and Resonance and Field Parameters", in Chem. Rev. 1991. 97, 165-195, entnommen werden, die hierin in ihrer Gesamtheit eingearbeitet ist. Für die vorliegende Offenbarung von Bedeutung ist dabei jeweils ausschließlich der Wert $\sigma$ für die para-Position ($\sigma_p$), unabhängig davon, an welcher Position sich der zumindest eine Substituent $R^H$ letztlich befindet.

Beispiele bzw. Referenzbeispiele der Offenbarung sind Verbindungen der Tabelle 1, einschließlich ihrer pharmazeutisch verträglichen Salzverbindungen.

Tabelle 1

| Verb. | Formel | Lösungsmittel für Kristallisation | Schmelzpunkt °C | Bruttoformel | MS EI (m / z) ($M^+$) | Analyse Berechnet (B) % Gefunden (G) % | $^1H$ NMR (DMSO-$d_6$) $\delta$, ppm |
|---|---|---|---|---|---|---|---|
| CRCV-340 | 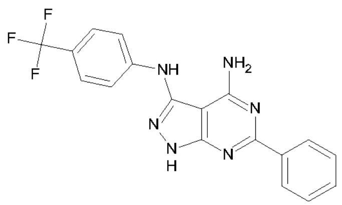 | THF, Toluol | - | $C_{18}H_{13}F_3 N_6$ | 370.3312 | B: C 58.3; H 3.54; N 22.69 | - |
| MS-112 (Referenz) | 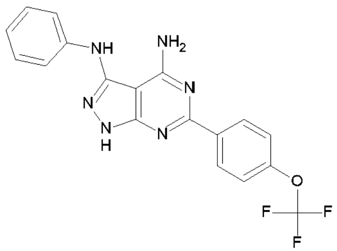 | EtOH | - | $C_{18}H_{13}F_3 N_6O$ | 386.3306 | B: C 55.96; H 3.39; N 21.75 | - |
| MS-115 (Referenz) | 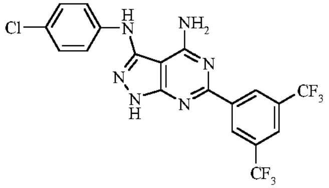 | DMF/EtOH | 272-74 | $C_{19}H_{11}Cl F_6N_6$ | 472.7831 | B: C 48.27; H 2.35; N 17.78 G: C 48.31; H 2.50; N 17.64 | - |
| MS-116 (Referenz) | 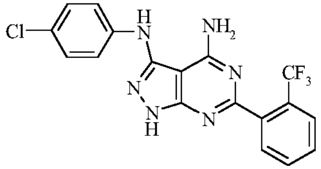 | Aktivkohle, EtOH | 271-73 | $C_{18}H_{12}Cl F_3N_6$ | 404.7847 | B: C 53.41; H 2.99; N 20.76 G: C 53.49; H 3.14; N 20.61 | 13.10 (1H, br.s., NH), 8.84 (1H, d, CH), 8.55 (1H, br.s., NH), 7.01-8.02 (9H, br.m, $NH_2$, 7CH) |

(fortgesetzt)

| Verb. | Formel | Lösungsmittel für Kristallisation | Schmelzpunkt °C | Bruttoformel | MS EI (m / z) ($M^+$) | Analyse Berechnet (B) % Gefunden (G) % | $^1$H NMR (DMSO-$d_6$) $\delta$, ppm |
|---|---|---|---|---|---|---|---|
| MS-117 (Referenz) | 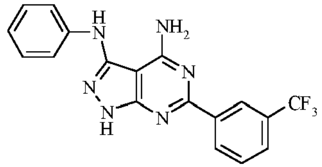 | EtOH | 230-32 | $C_{18}H_{13}F_3N_6$ | 370.3397 | B: C 58.38; H 3.54; N 22.69 G: C 58.61; H 3.67; N 22.45 | - |

Ebenfalls offenbart sind Pro-Pharmaka (Prodrugs) der Verbindungen, insbesondere jene, die sich durch einen Substituenten am Pyrazol-Heteroatom in Position 1 auszeichnen. Derartige Verbindungen werden *in vivo* zur 1H Pyrazol-Verbindung umgesetzt. Beispielhalber erwähnt seien Verbindungen, bei denen das Pyrazol-Heteroatom in Position 1 durch einen Imino(phenyl)methylSubstituenten substituiert ist, wie beispielsweise 1-[Imino(phenyl)methyl]-4-amino-3-(4-trifluormethyl-phenyl)amino-6-phenylpyrazolo[3,4-d]pyrimidin, auch bezeichnet mit dem IUPAC Namen 1-Benzylcarboximidoyl-6-phenyl-3-N-[4-(trifluormethyl)phenyl]-1H-pyrazolo[3,4-d]-pyrimidin-3,4-diamin. Hierbei handelt es sich um ein Nebenprodukt bei der Herstellung der oben bezeichneten Verbindung CRCV-340, das aus der Reaktion der Verbindung CRCV-340 mit einem Überschuss an Benzamidin in der Reaktionsmischung resultiert und folgende Formel aufweist:

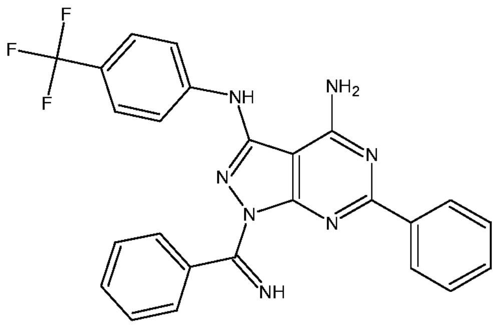

**CRCV-340-Prodrug**

Es wurde gefunden, dass diese Verbindungen (z.B. CRCV-340-Prodrug) *in vivo* sehr leicht zur Zielverbindung umgesetzt werden, derart dass im Serum im Wesentlichen nur noch der letztendliche Wirkstoff (z.B. CRCV-340) nachgewiesen werden kann. Weiterhin umfasst sind die aus den vorstehenden Verbindungen IIa und IIb hergestellten Salze, Solvate oder Solvate der Salze. Als Salze sind im Rahmen der vorliegenden Offenbarung physiologisch unbedenkliche Salze der Verbindungen der vorliegenden Offenbarung bevorzugt. Physiologisch unbedenkliche Salze der Verbindung der vorliegenden Offenbarung umfassen Additionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalinsulfonsäure, Essigsäure, Trifluoressigsäure, Propionsäure, Milchsäure, Weinsäure, Äpfelsäure, Citronensäure, Fumarsäure, Maleinsäure und Benzoesäure.

Die Offenbarung soll nachfolgend anhand von Syntheseverfahren, speziellen 4-Amino-3-phenylamino-6-phenylpyrazolo[3,4-d]pyrimidin-Derivaten der allgemeinen Formel (I) sowie deren Wirkung und Anwendung gegen Picornavirusinfektionen näher erläutert werden.

Abb. 1 zeigt ein allgemeines Schema zur Synthese von Pyrazolo[3,4-d]Pyrimidin 1 und schließt im ersten Schritt die Kondensation von [Bis(methylthio)methylen]malononitril 2 mit Phenylaminen 3 in Alkohol zu Phenylderivaten 4 ein. Letztere können jeweils isoliert und für weitere Reaktionen aufgereinigt werden oder direkt ohne Aufreinigung für nachfolgende Reaktion verwendet werden ("one-pot" Reaktion). Der darauf folgende Schritt besteht in der Wechselwirkung des Phenylderivats 4 mit Hydrazin oder Hydrazinderivaten. Die Reaktion verläuft unter Kochen über 1 bis 4 Stunden und führt zu einer hohen Ausbeute an Pyrazol 5. Der abschließende Schritt der Synthese von Pyrazolo [3,4-d]Pyrimidin 1 besteht in der Kondensation des Pyrazol 5 mit Phenylamidinen 6 in Gegenwart von Essigsäure, Trifluoressigsäure oder Natriumacetat.

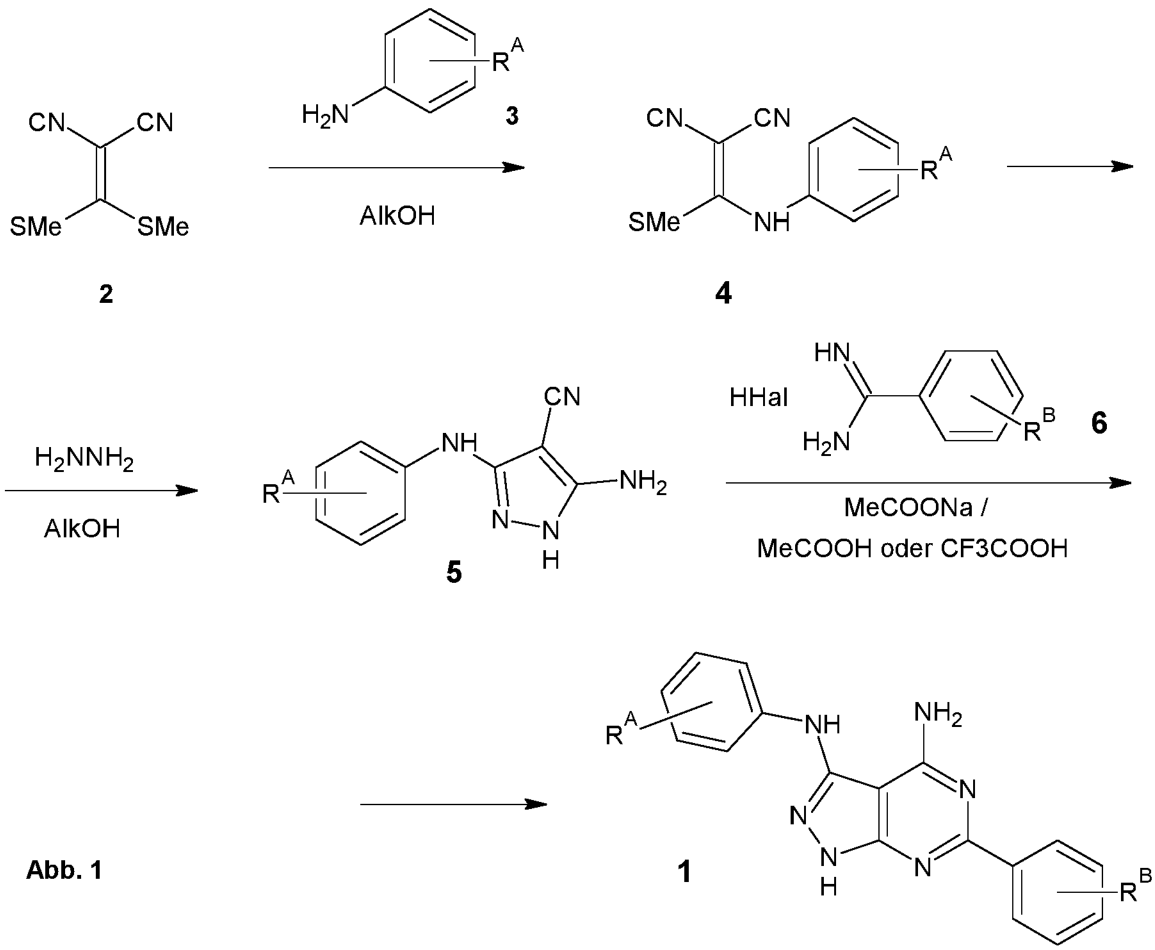


3

2

4

6

5

1

Abb. 1

Die Verbindungen können vorteilhaft dadurch erhalten werden, dass das Pyrazol (5) im letzten Syntheseschritt mit korrespondierendem Benzamidinhydrochlorid in der Anwesenheit eines Überschusses an Natriumacetat bei 200-220 °C in Abwesenheit von Lösungsmittel umgesetzt wird.

Alternativ können die Verbindungen dadurch erhalten werden, dass das Pyrazol (5) im letzten Syntheseschritt mit korrespondierendem Benzonitril (in großem Überschuss) unter Mikrowellenbestrahlung und unter Anwesenheit von Kalium-*tert*-butylat umgesetzt wird.

Eine alternative Synthesemethode besteht in der "one-pot" Reaktion von Malononitril mit Arylisothiocyanaten in Anwesenheit von Natriumhydrid und einer nachfolgenden Behandlung des Reaktionsgemisches mit Jodmethyl oder Dimethylsulfat. Dabei werden große Mengen an Enaminen gebildet. Auch hier ist wiederum die Kondensation von Pyrazol 5 mit Arylamidinen 6 in Gegenwart von Säure, wie Essigsäure oder Trifluoressigsäure, bzw. deren Salzen (Acetat) der abschließende Syntheseschritt zur Herstellung von Pyrazolo[3,4-d]Pyrimidin 1.

Es hat sich gezeigt, dass die Umsetzung des Pyrazols (5) zum Pyrazolo[3,4-d]Pyrimidin mit besonders hohen Ausbeuten durchgeführt werden kann, indem die Benzamidin-Komponente als freie Base verwendet und die Reaktion in polaren Lösungsmitteln durchgeführt wird. Ein Vorteil dieses Vorgehens besteht zusätzlich darin, dass der Anteil schwer abtrennbarer Nebenprodukte minimiert werden kann. Diese Reaktion lässt sich, ausgehend von substituierten 5-Amino-4-cyano-3-phenylamino-Pyrazolen (5) mit optional substituierten Benzamidinen als freie Base (6) zu 4-Amino-3-(phenylamino)-6-phenylpyrazolo[3,4-d]Pyrimidin (1) gemäß dem folgenden Reaktionsschema durchführen.

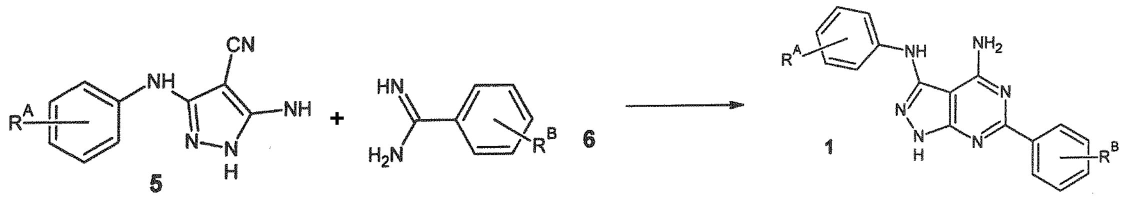

**Abb. 2**

Die Reste $R^A$ und $R^B$ sind Substituenten, wie sie vorstehend für $R^1$ definiert sind.

Während sich die wie vorstehend beschriebene Umsetzung insbesondere für die Herstellung von 4-Amino-3-phenylamino-6-phenylpyrazolo[3,4-d]pyrimidin-Derivaten der vorstehenden allgemeinen Formel (I) eignet, wobei mindestens ein Wasserstoffatom in mindestens einer der Phenylgruppen A und B durch einen Substituenten $R^H$ ersetzt ist, der eine Hammett-Konstante $\sigma_p$ > 0,23 aufweist, kann das Verfahren ebenso für die Herstellung von 4-Amino-3-phenylamino-6-phenylpyrazolo[3,4-d]pyrimidinen, die keinen solchen Substituenten aufweisen, d.h. in denen dieser Rest Wasserstoff oder einen Rest $R^1$, wie er vorstehend im Zusammenhang mit der Formel I definiert ist, darstellt.

Offenbart sind auch Reaktionen, wie vorstehend beschrieben, indem die Reste $R^A$ und $R^B$ voneinander unabhängig aus $NO_2$, CN, $CONR^3_2$, $COOR^3$, CHO, $CHONH_2$, einem Halogen, einem ungesättigten oder gesättigten linearen oder verzweigten aliphatischen Radikal (Alkylgruppe genannt) mit 1 bis 6 Kettengliedern, einem gesättigten oder ungesättigten linearen oder verzweigten Alkanolradikal (auch Alkoxygruppe genannt) mit 1 bis 6 Kettengliedern, $OR^3$, $SR^3$, $NR^3_2$, $SO_2NR^3$, Di- oder Trifluormethyl ausgewählt sein können und der Rest $R^3$ aus H, Methyl-, Ethyl-, Propyl- oder Butylgruppen bestehen kann. Sofern die Verbindungen der vorliegenden Offenbarung in tautomeren Formen vorkommen können, umfasst die vorliegende Offenbarung sämtliche tautomere Formen.

Die im vorstehenden Absatz genannten Substituenten haben, soweit nicht anders spezifiziert, die folgende Bedeutung:

Alkyl sowie die Alkylteile in Alkoxy stehen für geradkettige oder verzweigte Alkyle und umfassen, wenn nicht anders angegeben, (C1-C6)-Alkyl, insbesondere (C1-C4)-Alkyl, wie z.B. Methyl, Ethyl, Propyl, Isopropyl, Butyl, Isobutyl. Alkoxy steht vorzugsweise für einen geradkettigen oder verzweigten Alkoxyrest, insbesondere mit 1 bis 6, besonders bevorzugt 1 bis 4 und am meisten bevorzugt 1 bis 3 Kohlenstoffatomen. Beispielhaft und vorzugsweise seien genannt Methoxy, Ethoxy, N-Propoxy, Isopropoxy, t-Butoxy, n-Pentoxy und n-Hexoxy. Aryl steht für einen mono- bis tricyclischen aromatischen, carbocyclischen Rest mit in der Regel 6 bis 14 Kohlenstoffatomen. Beispielhaft und vorzugsweise ist Aryl ausgewählt aus Phenyl, Naphthyl und Phenantrenyl, besonders bevorzugt Phenyl. Halogen steht für Fluor, Chlor, Brom und Jod, bevorzugt Fluor und Chlor.

Es ist auch möglich mit diesem Verfahren allgemein Arylpyrazolo[3,4d]pyrimidin-Derivate herzustellen, wobei in diesem Fall das Phenylamidin 6 durch ein entsprechendes Arylamidin zu ersetzen ist. Insbesondere können anstelle der Phenylringe A und B auch Naphthyl, Pyridyl, Chinolyl, Pyrazinyl, Pyrimidyl, Pyrazolyl, Triazinyl, Imidazolyl, Furanyl, Thienyl im Endprodukt enthalten sein, wobei jedes Wasserstoffatom in jeder der vorstehend genannten Gruppe unabhängig voneinander durch einen Rest $R^1$, wie er vorstehend im Zusammenhang mit der Formel I definiert ist, ersetzt sein kann.

Die in Abb. 2 gezeigt Reaktion wird in inerten, polaren organischen Lösungsmitteln durchgeführt. Inerte polare organische Lösungsmittel sind beispielsweise Ether wie Diethylether, Methyl-tert.-butylether, 1,2-Dimethoxyethan, Glycoldiethylether oder Diethylenglycoldimethylether, cyclische Ether, wie Dioxan, Tetrahydrofuran, Kohlenwasserstoffe, wie Ethylbenzol, Xylol, Toluol oder Alkohole, wie Ethanol, Propanol, Butanol, Isobutanol und Isopropanol. Besonders reine Produkte werden bei Verwendung von n-Butanol als Lösungsmittel erhalten. n-Butanol ist vorzugsweise in einem Molverhältnis von 1 bis 10 , besonders bevorzugt 1,5 bis 3, bezogen auf den Anfangswert des Pyrazolderivats, einzusetzen.

Im Rahmen der vorliegenden Offenbarung hat es sich als besonders zweckmäßig herausgestellt, wenn die Benzamidine (6) in Grundform (als freier Basen) frisch zubereitet werden. Die Synthese erfolgt mit üblichen Methoden aus dem entsprechenden verfügbaren Salz. Es ist am besten, Benzamidine (6) in einem Molverhältnis 1 bis 1,5, ausgehend von den Pyrazolderivaten (5) einzusetzen.

Die Reaktion wird bei einer Temperatur von 60 bis 110°C, vorzugsweise 85 bis 95°C, im Laufe von 10-30 Stunden, vorzugsweise 18-20 Stunden, durchgeführt.

Die Reinigung der so erhaltenen Amino-3-(phenylamino)-6-phenylpyrazolo[3,4-d]Pyrimidine (1) erfolgt durch Umkristallisation. Hierfür wird vorzugsweise Tetrahydrofuran oder ein Gemisch von Tetrahydrofuran mit Wasser oder einem organischen Lösungsmittel, besonders bevorzugt mit Toluol verwendet. Alternativ dazu kann das Amino-3-(phenylamino)-6-phenylpyrazolo[3,4-d]Pyrimidin auch durch Ausfällen aus einer heißen Lösung in Tetrahydrofuran mit Wasser oder einem organischen Lösungsmittel, bevorzugt mit Toluol, gereinigt werden.

In den nachfolgenden Beispielen bzw. Referenzbeispielen sind spezielle Verbindungen der allgemeinen Formel IIa und IIb aufgelistet, welche bevorzugt für Anwendungen gegen Picornavirusinfektionen geeignet sind, wobei die Verbindungen in einer Lösung oder einer Suspension in einem pharmazeutisch akzeptablen wässrigen, organischen oder wässrig-organischen Medium für die lokale oder parenterale Anwendung durch intravenöse, subkutane oder intramuskuläre Injektion oder für die intranasale Verabreichung zubereitbar ist, oder sie sind in Form einer Tablette, Kapsel bzw. wässrigen Suspension mit konventionellem Träger für die orale Verabreichung oder als Suppositorium ausgebildet.

Die vorgestellten Verbindungen der Formel IIa und IIb können dabei in Dosierungen von 0,1 bis 1000 mg/kg Körpergewicht verwendet werden.

**Herstellung und Analyse der 4-Amino-3-phenylamino-6-phenylpyrazolo[3,4-d]pyrimidin-Derivate**

Die Strukturaufklärung der Verbindungen der vorliegenden Offenbarung erfolgte durch die Art der Synthese, Elementaranalysen, NMR - und Massenspektroskopie.

**Ausgangsmaterialien:**

Die 5-Amino-4-cyano-3-phenylaminopyrazole wurden gemäß dem in Abb. 1 dargestellten Verfahren sowie nach der Beschreibung von Tominaga Y et al. (J. Heterocycl. Chem., 1990, 27, 775-779), synthetisiert. Arylamidine werden gemäß dem bekannten Stand der Technik aus den entsprechenden Cyan-Ausgangsverbindungen synthetisiert (Boere, RT et al.: J. Organomet. Chem., 1987, 331, 161-167; Garigipati RS: Tetrahedron Lett., 1990, 31, 1969-1978; Dann O etal.: Justus Liebigs Ann. Chem., 1982, 1836-1839).

Beispiele, die nicht vom Umfang der beigefügten Ansprüche umfasst werden, sind nicht Teil der Erfindung.

**Referenzbeispiel 1: 4-Amino-3-phenylamino-6-phenylpyrazolo [3,4-d] pyrimidin** (3-N,6-diphenyl-2H-pyrazolo[3,4-d]pyrimidin-3,4-diamin)

Unter Rühren werden zu 2,3 g (11,5 mmol) 5-Amino-4-cyano-3-phenylaminopyrazol 3,0 g (17,24 mmol) Benzamidin Hydrochlorid Hydrat und 2,2 g (23,0 mmol) Natriumacetat zugegeben. Das Reaktionsgemisch wird für 30 min bei 220 °C erhitzt. Das resultierende Material wird mit 50 ml Wasser behandelt, gefiltert und mit 20 ml kaltem Methanol sowie 20 ml kaltem Ester gewaschen. Das Produkt wird mittels Kristallisation aus DMF/Wasser gereinigt.

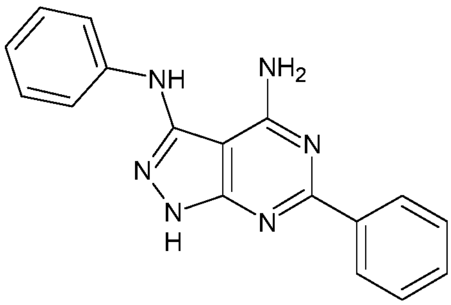

**Ref-1**

Hellgelber, fester kristalliner Stoff. Ausbeute 57 %. mp 253-5 °C. $R_f$(Chloroform - Methanol; 10/1) - 0.8 (Silikagel 60). MS m/z 302 ($M^+$).

**Referenzbeispiel** 2: **4-Amino-3-(phenylamino)-6-phenylpyrazolo[3,4-d]pyrimidin (alternative Herstellung)**

5,22 g Benzamidin Hydrochlorid, das zuvor 2,5 h lang bei 115°C getrocknet wurde, wird langsam zu einer Lösung von 1,74 g Natriummethylat in 100 ml Methanol gegeben und 30 min bei Raumtemperatur gerührt. Nach Abfiltrieren des anorganischen weißen Niederschlages wird 3,5 ml n-Butanol hinzu gegeben und das Volumen im Vakuum auf 3 ml reduziert. Der Rückstand ist ein weißliches Öl, und entspricht 4,0 g Benzamidin, das sofort im nächsten Reaktionsschritt verwendet wird.

5-Amino-4-cyano-3-(phenylamino)-pyrazol (6,0 g; Pulver Ref-1) wird in 10 ml n-Butanol gelöst und 4,0 g Benzamidin in 3 ml n-Butanol werden bei Raumtemperatur hinzu gegeben. Die Reaktion erfolgt im Laufe von 20 Stunden bei 85°C. Anschließend wird die Lösung abgekühl, der gelbe Niederschlag abfiltriert und mit 5 ml n-Butanol und 5 ml Toluol gewaschen.

Ausbeute: 68%

Eigenschaften: Schmelzpunkt: 265-267°C (Tetrahydrofuran); MS (m/z) 302 ($M^+$); $^1H$ NMR (DMSO-$d_6$): δ 12,38 (1H, s, NH), 8,30-8,36 (2H, q, 2 CH), 8,23 (1H, br. s., NH), 7,67 (2H, d, 2CH), 7,48 (2H, br. s., $NH_2$), 7,42 (3H, m, 3CH), 7,12 (2H, d, 2CH) und 6,98 (1H, m, CH) ppm; Elementaranalyse $C_{17}H_{14}N_6$: Berechnet %: C, 67,54; H, 4,67; N, 27,80; Gefunden %: C, 67,49; H, 4,53; N, 27,74.

**Referenzbeispiel 3: 4-Amino-3-(4-chlorphenyl)amino-6-phenylpyrazolo[3,4-d]pyrimidin** (3-N-(4-chlorphenyl)-6-phenyl-2H-pyrazolo[3,4-d]pyrimidin-3,4-diamin)

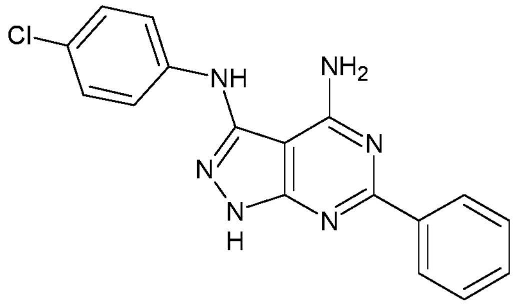

**Ref-3**

**Referenzbeispiel 4: 4-Amino-3-(3,4-difluorphenyl)amino-6-phenylpyrazolo[3,4-d]pyrimidin** (3-N-(3,4-difluorphenyl)-6-phenyl-2H-pyrazolo[3,4-d]pyrimidin-3,4-diamin)

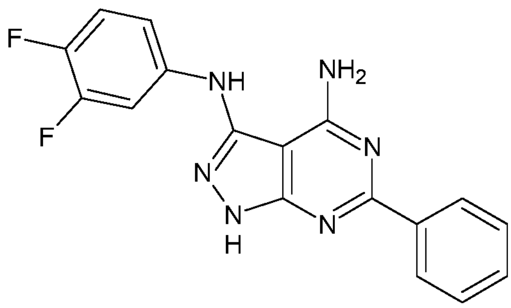

**Ref-4**

**Referenzbeispiel 5: 4-Amino-3-[(4-fluorophenyl)amino]-6-phenylpyrazolo[3,4-d]pyrimidin**

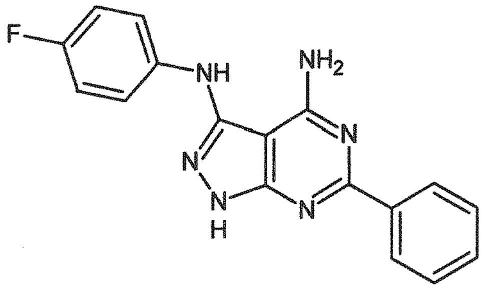

Ref-5

Die Verbindung Ref-5 wird auf die gleiche Weise, wie in Referenzbeispiel 2 dargestellt, hergestellt, wobei jedoch als Ausgangsstoff 5-Amino-4-cyano-3-[(4-fluorophenyl)amino)-pyrazol verwendet wurde. Dabei bildet sich ein hellgelber

kristalliner Niederschlag.

Ausbeute: 70%

Eigenschaften: Schmelzpunkt: 262-263°C (THF/Toluol); MS (m/z): 320 (M'); $^1$H NMR (DMSO-$d_6$): δ 12,69 (1H, s, NH), 8,33-8,41 (4H, m, 4CH), 8,18 (1H, br. s., NH), 7,58-7,65 (5H, m, $NH_2$, 3CH), 7,27-7,31 (2H, n, 2CH) ppm; Elementaranalyse $C_{17}H_{14}FN_6$: Berechnet %: C, 63,74; H, 4,09; N, 26,24; Gefunden %: C, 63,81; H, 4,11; N, 26,27.

**Referenzbeispiel 6: 4-Amino-3-[(3-fluorophenyl)amino]-6-phenylpyrazolo[3,4-d]pyrimidin**

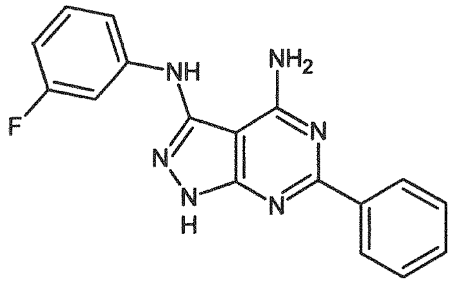

Ref-6

Die Verbindung Ref-6 wird auf die gleiche Weise, wie in Referenzbeispiel 2 erhalten, hergestellt, wobei jedoch als Ausgangskomponente 5-Amino-4-cyano-3-[(3-fluorophenyl)amino]-pyrazol verwendet wurde. Dabei bildet sich ein hellgelber kristalliner Niederschlag.

Ausbeute: 76%

Eigenschaften: Schmelzpunkt: 278-279°C (THF/Toluol); MS (m/z): 320 ($M^+$); $^1$H NMR (DMSO-$d_6$); δ 12,61 (1H, s, NH), 8,34-8,42 (2H, q, 2CH), 8,14 (1H, br. s., NH), 7,48 (2H, br. s., $NH_2$), 7,3-7,43 (6H, m, 6CH), 6,60 (1H, s, CH) ppm; Elementaranalyse für $C_{17}H_{14}FN_6$: Berechnet %: C, 63,74; H, 4,09; N, 26,24; Gefunden %: C, 63,81; H, 4,11; N, 26,27.

**Referenzbeispiel 7: 4-Amino-3-phenylamino-6-[4-(trifluormethoxy)-phenyl]pyrazolo[3,4-d]pyrimidin (MS-112)**

(3-N-phenyl-6-[4-(trifluormethoxy)phenyl]-2H-pyrazolo[3,4-d]pyrimidin-3,4-diamin)

Die Herstellung erfolgte wie im Referenzbeispiel 1 beschrieben, unter Verwendung der entsprechend substituierten Vorläuferverbindungen.

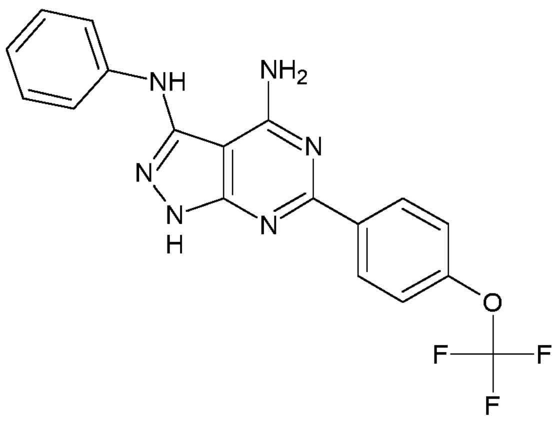

**MS-112 (Ref-7)**

**Referenzbeispiel 8: 4-Amino-6-phenyl-3-[(4-(trifluoromethoxy)-phenylamino]-pyrazolo[3,4-d]pyrimidin**

Diese Verbindung wurde ebenfalls analog der in Referenzbeispiel 2 dargestellten Reaktionsvorschrift hergestellt, wobei 5-Amino-4-cyano-3-[(4-trifluormethoxyphenyl)amino]pyrazol aus Ausgangsverbindung verwendet wurde. Dabei bildete sich ein weißer kristalliner Niederschlag.

Ausbeute: 68%

Eigenschaften: Schmelzpunkt: 260-262°C (Tetrahydrofuran/DMF); MS (m/z): 386 (M'); $^1$H NMR (DMSO-$d_6$): $\delta$ 12,56 (1H, s, NH), 8,82 (2H, q, 2CH), 8,16 (1H, br. s., NH), 7,48 (2H, br. s., $NH_2$), 7,3-7,43 (4H, m, $C_6H_5$), 7,05-7,11 (2H, 2 s, 2CH), 6,98 (1H, m, CH) ppm; Elementaranalyse $C_{18}H_{13}F_3N_6O$: Berechnet %: C, 55,96; H, 3,39; N, 21,75; Gefunden %: C, 56,07; H, 3,36; N, 21,61.

**Beispiel 1: 4-Amino-3-(4-trifluormethyl-phenyl)amino-6-phenylpyrazolo[3,4-d]pyrimidin (CRCV-340)**

(6-phenyl-3-N-[4-(trifluormethyl)phenyl]-2H-pyrazolo[3,4-d]pyrimidin-3,4-diamin)

Die Herstellung erfolgte wie im Referenzbeispiel 1 beschrieben, unter Verwendung der entsprechend substituierten Vorläuferverbindungen.

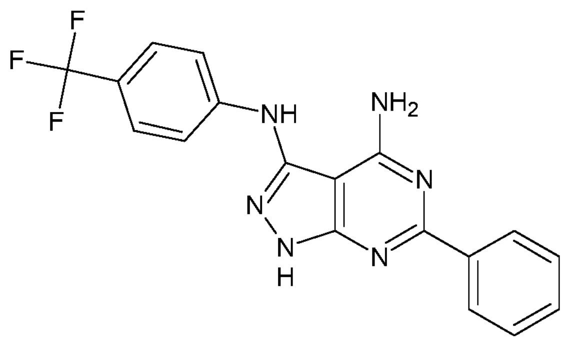

**CRCV-340**

**Beispiel 2: 4-Amino-6-phenyl-3-[(4-(trifluoromethyl)-phenyl]amino-pyrazolo[3,4-d]pyrimidin**

Die Verbindung CRCV-340 wurde ebenfalls analog der in Referenzbeispiel 2 dargestellten Reaktionsvorschrift hergestellt. Dabei bildet sich ein hellgelber kristalliner Niederschlag.
Ausbeute: 58%
Eigenschaften: Schmelzpunkt: 313-314°C (THF/Toluol); MS (m/z): 370 ($M^+$); $^1$H NMR (DMSO-$d_6$): $\delta$ 12,77 (1H, s, NH), 8,91 (1H, s, NH), 8,47 (2H, s, $NH_2$), 7,81, 7,79, 7,63, 7,58 und 7,47 (9H, m, $C_6H_4$ und $C_6H_5$) ppm; Elementaranalyse $C_{18}H_{13}F_3N_6$: Berechnet %: C, 58,38; H, 3,54; N, 22,69; Gefunden %: C, 58,41; H, 3,58; N, 22,74; HPLC: 99,30% (Säule Luna C18 (2), Acetonitril/Wasser - 90:10, Durchfluss von 0,6 ml/min, UV 254 nm; $t_R$ = 5,3 min)

**Beispiel 3: Reinigung von 4-Amino-6-phenyl-3-[(4-(trifluoromethyl)-phenyl]amino-pyrazolo[3,4-d]pyrimidin**

50 g trockenes 4-Amino-6-phenyl-3-[(4-trifluoromethyl)-phenyl]amino-pyrazolo[3,4-d]pyrimidin, synthetisiert nach dem in Referenzbeispiel 2 beschriebenen Verfahren, werden unter Erwärmen in 300 ml von THF gelöst. Diese Lösung wird mit 300 ml kaltem Toluol behandelt. Die resultierende Lösung wird bei einer Temperatur unter 0°C im Laufe von 6 Stunden in einem Gefrierschrank gehalten. 36 g 4-Amino-3-(4-trifluorophenyl)amino-6-phenylpyrazolo[3,4-d]pyrimidin in gelben Kristallen werden durch Filtration erhalten.
Schmelzpunkt: 313-314°C (THF/DMF)
HPLC: 99,16% (Säule Luna C18(2), Acetonitril/Wasser -90:10, Durchfluss von 0,6 ml/min, UV 254 nm; $t_R$ = 5,3 min)

**ADMET-Studien zum Metabolismus von OBR 5-340 *in vitro***

Ziel: Überprüfung der Aufnahme (Absorption), Verteilung (Distribution), des biochemischen Um- bzw. Abbaus (Metabolismus), der Ausscheidung (Exkretion) sowie Toxizität (abgekürzt: ADMET) von OBR 5-340 *in vitro,* sowie *In vitro*-Untersuchungen zur Plasmaproteinbindung und zur Stabilität im Plasma und in Lebermikrosomen.
Untersuchungen: Die durchgeführten *In vitro*-Tests mit CRCV-340 sowie die erhaltenen Daten sind im Vergleich zu Ref-2 in Tabelle 2 zusammengefasst.

Tabelle 2: Zusammenfassung der Ergebnisse aus den ADMET-Studien für CRCV-340 im Vergleich zu Ref-2.

| Untersuchungsparameter | Ref-2 | CRCV-340 |
|---|---|---|
| Bindung an Plasmaprotein | 99,5 % | 96,7 % |

(fortgesetzt)

| Untersuchungsparameter | Ref-2 | CRCV-340 |
|---|---|---|
| Freisetzung von Plasmaprotein | 61,0 % | 122,3 % |
| Plasmastabilität | 117,0 % | 100,0 % |
| Stabilität in Lebermikrosomen | 32,0 % | 112,0 % |

Zusammenfassung: Die Substanz CRCV-340 zeichnete sich durch eine bessere Stabilität in Lebermikrosomen sowie eine stärkere Freisetzung von Plasmaprotein im Vergleich zu Ref-2 aus.

**Untersuchung der Pharmakokinetik von CRCV-340 in der Maus**

Ziel: Erhebung pharmakokinetischer Daten zu CRCV-340.

Untersuchungen: Substanz und Referenzsubstanz wurden jeweils einmalig in einer Konzentration von 100 mg/kg KG, in 0,5 ml einer 10%igen Cremophorlösung *per os* den Mäusen appliziert. Nach 0,5, 1, 2, 3, 4, 5, 6 und 7 h wurde das Serum gewonnen und mittels HPLC-Analyse die Konzentrationen von CRCV-340, Ref-2 und Ref-3 im Plasma der Mäuse bestimmt.

Anhand der mittels HPLC-festgestellten Substanzkonzentrationen im Blutplasma wurden die pharmakokinetischen Parameter der Substanzen mit dem Computerprogramm ESTRIP berechnet (Tabelle 3).

Tabelle 3: Pharmakokinetische Daten nach einmaliger, intragastraler Applikation von 100 mg/kg CRCV-340, Ref-2 und Ref-3 in Mäuse.

| Substanz | C max (ng/ml) | T max (h) | MRT (h) | $T_{1/2}$ (h) | $K_{el}$ ($h^{-1}$) | CL ($ml \times h/kg$) | $V_d$ (ml/kg) | $AUC_{0-t}$ ($ng \times ml/h$) | $AUC_{0-\infty}$ ($ng \times ml/h$) | $C_{max}/A\ UC_{0-t}$ ($h^{-1}$) |
|---|---|---|---|---|---|---|---|---|---|---|
| Ref-2 | 1295,4 | 1,0 | 2,14 | 2,96 | 0,23 | 36,6 | 156,7 | 2354,5 | 2732,1 | 0,55 |
| Ref-3 | 424,5 | 0,5 | 2,44 | 1,47 | 0,55 | 91,8 | 168,1 | 1085,7 | 1089,4 | 0,39 |
| CRCV-340 | 1254,4 | 3,0 | 3,54 | 3,54 | 0,18 | 10,4 | 52,7 | 6418,3 | 9598,5 | 0,20 |

| | |
|---|---|
| $C_{max}$ | Maximalkonzentration im Blut |
| $T_{max}$ | Zeit bis zum Erreichen der Maximalkonzentration im Blut |
| MRT | Mittlere Verweilzeit im Blut (mean residence time) |
| $T_{1/2}$ | Halbwertszeit |
| $K_{el}$ | Eliminationskonstante |
| CL | Beseitigung (clearance) |
| $V_d$ | Verteilungsvolumen (volume of distribution) |
| AUC | Fläche unter der Kurve (area under curve) |
| $C_{max}$/AUC | Absorptionsgeschwindigkeit von Magen zum Blut |

Ergebnis: Die Untersuchungen zur Pharmakokinetik in der Maus ergaben für CRCV-340 eine deutlich bessere Bioverfügbarkeit als für Ref-2 und Ref-3.

**Untersuchungen zur akuten Toxizität von CRCV-340 in der Maus**

Ziel: Bestimmung der 50 % letalen Dosis von CRCV-340

Untersuchungen: Die akute Toxizität der Substanz CRCV-340 wurde in 19,5 - 20,5 g schweren Mäusen überprüft, denen *per* os 40, 60, 80 bzw. 120 mg Substanz/Maus (je 5 Mäuse pro Substanz und Konzentration) verabreicht wurden. Dies entspricht einer Dosis von ca. 2g, 3g, 4g bzw. 6g pro Kilogramm Körpergewicht. Die im Ergebnis beobachteten toxischen Effekte korrelierten mit der applizierten Substanzdosis. Bei den 40 und 60 mg/Maus-Dosierungen wurden bis zu 3 h nach Substanzgabe Koordinationsstörungen und Hyperaktivität als Nebenwirkungen beobachtet. Nach Applikation der zwei höheren Dosierungen kamen Atembeschwerden, aggressives Verhalten und Hyperkinesie dazu. Für CRCV-340 wurde eine $LD_{50}$ von 3120 mg/kg bestimmt (Tabelle 4).

Tabelle 4: Ergebnisse der Untersuchungen zur akuten Toxizität der Substanz CRCV-340 nach einmaliger peroraler Applikation in der Maus

| Dosis | Tote/Überlebende 72 h nach Applikation von | |
|---|---|---|
| | Ref-2 | CRCV-340 |
| 40 mg/Maus | 0/5 | 1/5 |
| 60 mg/Maus | 1/5 | 1/5 |
| 80 mg/Maus | 3/5 | 3/5 |
| 120 mg/Maus | 4/5 | 5/5 |
| $LD_{50}$ | 78,6 mg/Maus (3930 mg/kg) | 62,4 mg/Maus (3120 mg/kg) |

Ergebnis: Die erhaltenen Daten zur Überlebensrate belegen eine sehr gute Verträglichkeit beider Testsubstanzen.

**Langzeituntersuchungen zur Toxizität in der Maus**

Ziel: Feststellung der subakuten Toxizität in der Maus, um mögliche Nebenwirkungen z.B. auf das Allgemeinbefinden, Gewicht, die inneren Organe und den Stoffwechsel, die bei einer subakuten Gabe von CRCV-340 über 28 Tage auftreten könnten, aufzudecken.

Untersuchungen: Dazu erhielten ca. 20 g schwere männlich und weibliche Mäuse 12,5, 50 oder 200 mg/kg der Substanz CRCV-340 in einer Cremophorformulierung bzw. die Kontrollgruppe Cremophor einmal täglich über 28 Tage intragastral über eine Sonde verabreicht. Im gesamten Versuchszeitraum wurde der Zustand des Fells und der Schleimhäute, der Exkrete und das Allgemeinbefinden bewertet. Die Protokollierung des Gewichts erfolgte an den Tagen 7, 14, 21 und 28. Ca. 24 h nach der letzten Substanzgabe wurde das Orientierungsvermögen der Tiere untersucht. Im Serum wurden folgende biochemische Parameter analysiert: Proteingehalt, Harnstoff, Creatinin, die Serumaktivität von Aspartataminotransferase, Alaninaminotransferase und alkalischer Phosphatase. Die Hämoglobinkonzentration, Hämatokrit, die Anzahl der roten und weißen Blutzellen sowie der Blutblättchen wurde im Blutausstrich bestimmt. Zum Versuchsende wurden die Mäuse unter Narkose getötet und seziert, der Zustand der inneren Organe makroskopisch eingeschätzt, das Organgewicht bestimmt und Proben für nachfolgende histologische Untersuchungen in Formalin eingelegt.

Ergebniszusammenfassung: Die Ergebnisse belegen eine sehr gute Verträglichkeit der untersuchten Substanzkonzentrationen. Weder das Allgemeinbefinden der Tiere, ihr Fell, die Exkrete noch das Orientierungsvermögen

bzw. die Beweglichkeit veränderten sich infolge der Substanzgabe. Auch in Bezug auf die Körpergewichtsdynamik unterschieden sich die CRCV-340-behandelten Tiere nicht von der Kontrollgruppe. Es ließen sich keine Unterschiede zwischen den substanzbehandelten und vehikelbehandelten Versuchsgruppen im Blutausstrich, bei den biochemischen Blutparametern feststellen. Gleiches gilt für die makroskopische Begutachtung der inneren Organe (Leber, Niere, Herz, Lunge, Milz, Pankreas und Hoden), die Organgewichte sowie die Ergebnisse der histologischen Untersuchungen von Herz-, Lungen-, Leber-, Milz-, Thymus-, Pankreas-, Nieren-, Drüsen-, Hoden-, Magen- und Darmgewebsproben (Ergebnisse nicht dargestellt).

**Bestimmung des antiviralen Spektrums gegen Rhinoviren**

Ziel: Bestimmung des antiviralen Wirkspektrums von CRCV-340 gegenüber 50 verschiedenen humanen Rhinovirus-Serotypen und CVB3-Patientenisolaten.

Untersuchungen: Die 50 HRV-Serotypen und 20 klinischen CVB3-Isolate wurden in HeLa-, HeLa Wis- und/oder LF-Zellen vermehrt, ihr Titer bestimmt und mittels Sequenzierung der Serotyp überprüft. Danach wurden zpE (zytopathischer Effekt)-Hemmtests mit den HRV bzw. Plaque-Reduktions-Tests mit den CVB3 Isolaten etabliert. In den entsprechenden antiviralen Tests erfolgten Dosis-Wirkungs-Untersuchungen mit Ref-3 sowie CRCV-340. Eine Übersicht zu den ermittelten mittleren 50 %-Hemmkonzentrationen geben die Tabellen 5 und 6.

Tabelle 5: Übersicht zum Wirkspektrum von Ref-3 und CRCV-340 gegenüber HRV. Zusammengefasst wurden die Mittelwerte und Standardabweichungen für alle 50 untersuchten Serotypen sowie für die 45 Pleconaril-sensitiven und 5 Pleconaril-resistenten Serotypen getrennt.

| Substanz | $IC_{50}$[$\mu$M] alle HRV (n = 50) | | $IC_{50}$[$\mu$M] Pleconaril-sensitive HRV (n = 45) | | $IC_{50}$[$\mu$M] Pleconaril-resistente HRV (n = 5) | | Anzahl der sensitiven HRV |
|---|---|---|---|---|---|---|---|
| | mean | S. D. | mean | S. D | mean | S. D. | |
| Ref-3 | 16,11 | 11,94 | 18,52 | 10,88 | 0,07 | 0,01 | 23 |
| CRCV-340 | 25,73 | 21,49 | 26,09 | 18,98 | 22,62 | 40,69 | 49 |

Tabelle 6: Übersicht zum Wirkspektrum von Ref-3 und CRCV-340 gegenüber den klinischen CVB3-Isolaten. Zusammengefasst wurden die Mittelwerte und Standardabweichungen für alle 20 untersuchten Isolate sowie für die 19 Pleconaril-sensitiven Isolate und das eine Pleconarilresistente Serotypen getrennt.

| Substanz | $IC_{50}$[$\mu$M] alle CVB3 (n = 20) | | $IC_{50}$[$\mu$M] Pleconaril-sensitive CVB3 (n = 19) | | $IC_{50}$[$\mu$M] Pleconaril-resistente CVB3 (n = 1) | | Anzahl der sensitiven CVB3 |
|---|---|---|---|---|---|---|---|
| | mean | S. D. | mean | S. D | mean | S. D. | |
| Ref-3 | 0,68 | 0,90 | 0,71 | 0,91 | 0,13 | | 20 |
| CRCV-340 | 4,97 | 4,05 | 5,16 | 4,10 | 1,39 | | 18* |

* maximal getestete Konzentration 13,5 $\mu$M

Ergebniszusammenfassung: Das Wirkspektrum von CRCV-340 gegenüber HRV fiel im Vergleich zu Ref-3 deutlich breiter aus.

**Untersuchungen zur antiviralen Wirkung in vivo im Mausmodell der CVB3-induzierten chronischen Myokarditis**

Ziel: Bestätigung der antiviralen Wirkung von CRCV-340 im Mausmodell

Untersuchungen: Die Wirkung des CRCV-340 wurde im Modell der CVB 3-induzierten Myokarditis in 8-Wochen-alten männlichen NMRI Mäusen untersucht. Dazu erhielten CVB3 31-1-93 bzw. CVB3 H3-infizierte Tiere ein- oder zweimal täglich 100 mg/kg der Substanz in 50 % oder 20 % PEG-400 in 1 % CMC in Wasser (Placebo) über 7 Tage verabreicht. Parameter zur Bewertung des therapeutischen Effekts schlossen Veränderungen im Körpergewicht, Allgemeinbefinden, Virustiter in Herz- und Pankreasgewebe sowie histopathologische Veränderungen im Herz und Pankreas ein. Scheininfizierte, Placebo-behandelte bzw. CRCV-340-behandelte Tiere dienten als Negativkontrolle und infizierte,

Placebo- bzw. Pleconaril-behandelte Tiere als Positivkontrolle im Infektionsverlauf.

Ergebniszusammenfassung: Im Gegensatz zu Placebo und Pleconaril wirkte CRCV-340 im Modell der CVB3 31-1-93-induzierten chronischen Myokarditis in NMRI-Mäusen. Dabei waren klinisch und statistisch signifikante Effekte für alle untersuchten Parameter (Körpergewicht, Allgemeinbefinden, die Virustiter im Herz- und Pankreasgewebe am Tag 7 p.i., die Histopathologie im Herz- und Pankreas am Tag 7 und 21 p.i.) zu beobachten.

**Studien zur in vivo-Wirksamkeit von CRCV-340 im letalen Mausmodell im Vergleich zur Referenzsubstanz Pleconaril**

Ziel: Verifizierung der antiviralen Wirkung von CRCV-340 im letalen Mausmodell

Untersuchungen: Die Wirkung des Entwicklungskandidaten CRCV-340 wurde nach Austestung der Infektionsdosis im Modell der CVB 3-induzierten Myokarditis in 6 - 7 Wochen alten männlichen BALB/c Mäusen untersucht. Dazu erhielten CVB3 31-1-93 bzw. CVB3 H3-infizierte Tiere zweimal täglich 100 mg/kg der Substanz in 20 % PEG-400 in 1 % CMC in Wasser (Placebo) über 7 Tage verabreicht. Veränderungen im Körpergewicht, Allgemeinbefinden und die über den Surrogatmarker 25 % Körpergewichtsverlust definierte Letalität dienten als Parameter zur Bewertung des therapeutischen Effekts. Scheininfizierte, Placebo-behandelte bzw. CRCV-340-behandelte Tiere stellten die Negativkontrolle und infizierte, Placebo- bzw. Pleconaril-behandelte Tiere die Positivkontrolle im Infektionsverlauf dar.

Ergebniszusammenfassung: Im Gegensatz zu Placebo und Pleconaril wirkte CRCV-340 im letalen BALB/c-Mausmodell nach Infektion mit CVB3 31-1-93. Dabei waren klinisch und statistisch signifikante Effekte für alle untersuchten Parameter (Körpergewicht, Allgemeinbefinden sowie die Letalität) zu beobachten.

## Patentansprüche

1. 4-Amino-3-phenylamino-6-phenylpyrazolo[3,4-d]pyrimidin-Derivate der Formel

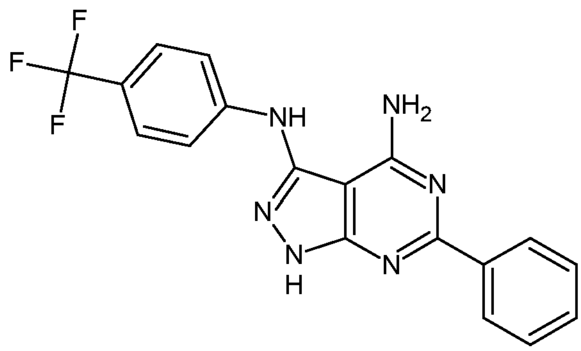

(CRCV-340).

2. Pharmazeutische Zusammensetzung, enthaltend ein 4-Amino-3-phenylamino-6-phenylpyrazolo[3,4-d]pyrimidin-Derivat nach Anspruch 1.

3. Pharmazeutische Zusammensetzung nach Anspruch 2, ferner enthaltend einen pharmazeutisch akzeptablen Träger.

4. Pharmazeutische Zusammensetzung nach Anspruch 2 oder 3, ferner enthaltend einen oder mehrere weitere Wirkstoffe.

5. Pharmazeutische Zusammensetzung nach Anspruch 4, wobei der eine oder die mehreren weiteren Wirkstoffe antivirale Wirkstoffe sind, bevorzugt Wirkstoffe gegen Picornaviren.

6. Verfahren zur Herstellung einer pharmazeutischen Zusammensetzung nach einem der Ansprüche 2-5, umfassend Formulieren eines 4-Amino-3-phenylamino-6-phenylpyrazolo[3,4-d]pyrimidin-Derivats nach Anspruch 1 mit einem pharmazeutisch akzeptablen Träger.

7. 4-Amino-3-phenylamino-6-phenylpyrazolo[3,4-d]pyrimidin-Derivate nach Anspruch 1 zur Verwendung als Medikament.

8. 4-Amino-3-phenylamino-6-phenylpyrazolo[3,4-d]pyrimidin-Derivate nach Anspruch 1 zur Verwendung bei der prophylaktischen oder therapeutischen Behandlung von Virusinfektionen.

9. 4-Amino-3-phenylamino-6-phenylpyrazolo[3,4-d]pyrimidin-Derivate zur Verwendung nach Anspruch 8, **dadurch gekennzeichnet, dass** die Virusinfektionen Picornavirusinfektionen sind.

10. Verfahren zur Herstellung eines 4-Amino-3-phenylamino-6-phenylpyrazolo[3,4-d]pyrimidin-Derivats nach Anspruch 1, umfassend die Umsetzung eines 5-Amino-4-Cyano-3-phenylaminopyrazols mit der freien Base eines Benzamidins in einem polaren organischen Lösungsmittel, bevorzugt in n-Butanol.

11. Verfahren gemäß Anspruch 10, wobei das erhaltene 4-Amino-3-phenylamino-6-phenylpyrazolo[3,4-d]pyrimidin-Derivat anschließend durch Umkristallisation aus Tetrahydrofuran oder einem Gemisch davon mit Wasser oder einem organischen Lösungsmittel oder durch Ausfällen aus einer heißen Lösung in Tetrahydrofuran mit Wasser oder einem organischen Lösungsmittel, gereinigt wird, wobei das organische Lösungsmittel bevorzugt Toluol ist.

**Claims**

1. 4-Amino-3-phenylamino-6-phenylpyrazolo[3,4-d]pyrimidine derivatives of formula

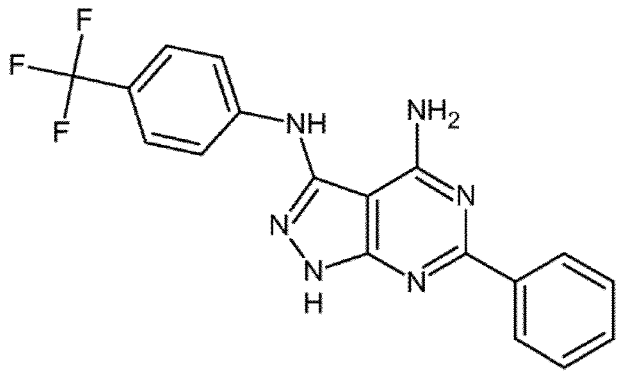

(CRCV-340).

2. A pharmaceutical composition containing a 4-amino-3-phenylamino-6-phenylpyrazolo[3,4-d]pyrimidine derivative according to claim 1.

3. The pharmaceutical composition according to claim 2, further containing a pharmaceutically acceptable carrier.

4. The pharmaceutical composition according to claim 2 or 3, further containing one or more further active ingredients.

5. The pharmaceutical composition according to claim 4, wherein the one or more further active ingredients are antiviral active ingredients, preferably active ingredients against picornaviruses.

6. A process for the preparation of a pharmaceutical composition according to any one of claims 2-5, comprising formulating a 4-amino-3-phenylamino-6-phenylpyrazolo[3,4-d]pyrimidine derivative according to claim 1 with a pharmaceutically acceptable carrier.

7. The 4-amino-3-phenylamino-6-phenylpyrazolo[3,4-d]pyrimidine derivatives according to claim 1 for use as a medicament.

8. The 4-amino-3-phenylamino-6-phenylpyrazolo[3,4-d]pyrimidine derivatives according to claim 1 for use in the prophylactic or therapeutic treatment of viral infections.

9. The 4-amino-3-phenylamino-6-phenylpyrazolo[3,4-d]pyrimidine derivatives for use according to claim 8, **characterised in that** the virus infections are picornavirus infections.

10. A process for the preparation of a 4-amino-3-phenylamino-6-phenylpyrazolo[3,4-d]pyrimidine derivative according

to claim 1, comprising reacting a 5-amino-4-cyano-3-phenylaminopyrazole with the free base of a benzamidine in a polar organic solvent, preferably in n-butanol.

**11.** The process according to claim 10, wherein the 4-amino-3-phenylamino-6-phenylpyrazolo[3,4-d]pyrimidine derivative obtained is subsequently purified by recrystallisation from tetrahydrofuran or a mixture thereof with water or an organic solvent or by precipitation from a hot solution in tetrahydrofuran with water or an organic solvent, the organic solvent preferably being toluene.

## Revendications

**1.** Dérivés de 4-amino-3-phénylamino-6-phénylpyrazolo[3,4-d]pyrimidine de la formule

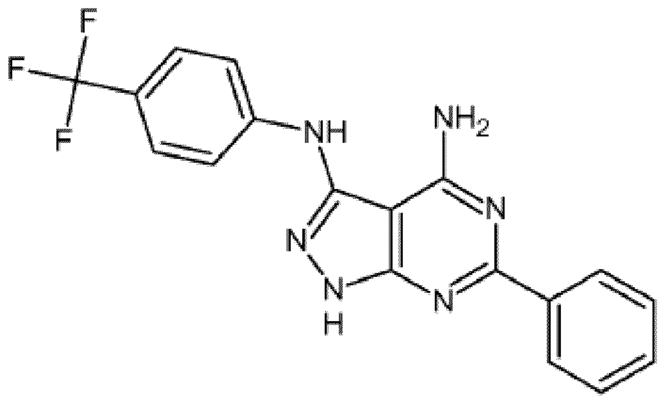

(CRCV-340).

**2.** Composition pharmaceutique contenant un dérivé de 4-amino-3-phénylamino-6-phénylpyrazolo[3,4-d]pyrimidine selon la revendication 1.

**3.** Composition pharmaceutique selon la revendication 2, contenant en outre un support pharmaceutiquement acceptable.

**4.** Composition pharmaceutique selon la revendication 2 ou 3, contenant en outre un ou plusieurs autres principes actifs.

**5.** Composition pharmaceutique selon la revendication 4, dans laquelle le ou les plusieurs autres principes actifs sont des principes actifs antiviraux, de manière préférée des principes actifs contre des picornavirus.

**6.** Procédé pour la manufacture d’une composition pharmaceutique selon l’une quelconque des revendications 2 - 5, comprenant la formulation d’un dérivé de 4-amino-3-phénylamino-6-phénylpyrazolo[3,4-d]pyrimidine selon la revendication 1 avec un support pharmaceutiquement acceptable.

**7.** Dérivés de 4-amino-3-phénylamino-6-phénylpyrazolo[3,4-d]pyrimidine selon la revendication 1 destinés à être utilisés en tant que médicament.

**8.** Dérivés de 4-amino-3-phénylamino-6-phénylpyrazolo[3,4-d]pyrimidine selon la revendication 1 destinés à être utilisés lors du traitement prophylactique ou thérapeutique d’infections virales.

**9.** Dérivés de 4-amino-3-phénylamino-6-phénylpyrazolo[3,4-d]pyrimidine destinés à être utilisés selon la revendication 8, **caractérisés en ce que** les infections virales sont des infections par des picornaviridés.

**10.** Procédé pour la manufacture d’un dérivé de 4-amino-3-phénylamino-6-phénylpyrazolo[3,4-d]pyrimidine selon la revendication 1, comprenant la réaction d’un 5-amino-4-cyano-3-phénylaminepyrazole avec la base libre d’une benzamidine dans un solvant organique polaire, de manière préférée dans du n-butanol.

**11.** Procédé selon la revendication 10, dans lequel le dérivé de 4-amino-3-phénylamino-6-phénylpyrazolo[3,4-d]pyrimidine obtenu est ensuite purifié par recristallisation à partir de tétrahydrofurane ou d’un mélange de celui-ci avec

de l'eau ou avec un solvant organique ou par précipitation à partir d'une solution chaude dans du tétrahydrofurane avec de l'eau ou avec un solvant organique, dans lequel le solvant organique est de manière préférée du toluène.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 674642 A
- EP 691128 A
- EP 496617 A
- US 4904666 A
- US 2965643 A
- US 3600389 A
- WO 0043394 A
- EP 2049540 A
- WO 2007147401 A
- WO 9413677 A


### In der Beschreibung aufgeführte Nicht-Patentliteratur


- **MELNICK J et al.** Virology. Lippincott-Raven Publishers, 1996, 655-712
- **COUCH RB et al.** Fields Virology. Lippincott-Raven, 1996, 713-35
- **ROTBART HA.** *Antiviral Res,* 2002, vol. 53 (2), 83-98
- **CROTTY S et al.** *Nat Med,* 2000, vol. 6 (12), 1375-9
- **HEINZ BA ; VANCE LM.** *J Virol,* 1995, vol. 69 (7), 4189-97
- **MILLER FD et al.** *Antimicrob Agents Chemother,* 1985, vol. 27 (1), 102-6
- **PATTICK AK et al.** *Antimicrobila Agents Chemother,* 1999, vol. 43 (10), 2444-50
- **DIANA GD.** *Curr Med Chem,* 2003, vol. 2, 1-12
- **PEVEAR DC et al.** *Antimicrob Agents Chemother,* 1999, vol. 43 (9), 2109-15
- **MCKINLAY MA et al.** *Annu Rev Microbiol,* 1992, vol. 46, 635-54
- **LEDFORD RM et al.** *J Virol,* 2004, vol. 78 (7), 3663-74
- **GROARKE JM et al.** *J Infect Dis,* 1999, vol. 179 (6), 1538-41
- **ABZUG MJ et al.** *Pediatr Infect Dis J,* 2003, vol. 22, 335-41
- **HAYDEN FG et al.** *Antivir Ther,* 2002, vol. 7, 53-65
- **HAYDEN FG et al.** *Clin Infect Dis,* 2003, vol. 36, 1523-32
- *J. Heterocyc. Chem.,* 1982, vol. 19, 1565
- **HANSCH et al.** A Survey of Hammett Substituent Constants and Resonance and Field Parameters. *Chem. Rev.,* 1991, vol. 97, 165-195
- **TOMINAGA Y et al.** *J. Heterocycl. Chem.,* 1990, vol. 27, 775-779
- **BOERE, RT et al.** *J. Organomet. Chem.,* 1987, vol. 331, 161-167
- **GARIGIPATI RS.** *Tetrahedron Lett.,* 1990, vol. 31, 1969-1978
- **DANN O et al.** *Justus Liebigs Ann. Chem.,* 1982, 1836-1839